Europäisches Patentamt

**European Patent Office**

**Office européen des brevets**

(11) Publication number: **0 370 581**
**A1**

## EUROPEAN PATENT APPLICATION

(21) Application number: 89202951.3

(22) Date of filing: 21.11.89

(51) Int. Cl.5: **C12N 15/00, A61K 37/00, A61K 39/00**

(30) Priority: **25.11.88 NL 8802902**

(43) Date of publication of application:
**30.05.90 Bulletin 90/22**

(84) Designated Contracting States:
**AT BE CH DE ES FR GB GR IT LI NL SE**

(71) Applicant: **AKZO N.V.**
**Velperweg 76**
**NL-6824 BM Arnhem(NL)**

(72) Inventor: **van Wezenbeek, Petrus Maria Gerardus Franciscus**
**Gewandeweg 44**
**NL-5345 HN Oss(NL)**
Inventor: **Bos, Ebo Sybren**
**De Vriesstraat 8**
**NL-5344 JA Oss(NL)**

(74) Representative: **Hermans, Franciscus G.M. et al**
**Patent Department AKZO N.V. Pharma Division P.O. Box 20**
**NL-5340 BH Oss(NL)**

(54) **Anti-hCG antibodies.**

(57) The invention relates to a polypeptide with binding-activity for hCG, containing at least one of the polypeptide fragments:

(i) ARG-ALA-SER-GLU-SER-VAL-ASP-ASN-ASN-GLY-ILE-SER-PHE-MET-ASN;

(ii) ALA-ALA-SER-ILE-GLN-GLY-SER;

(iii) GLN-GLN-ILE-LYS-GLU-VAL-PRO-PRO-THR;

(iv) ASP-TYR-ASN-MET-HIS;

(v) TYR-ILE-TYR-PRO-TYR-SER-GLY-PRO-THR-GLY-TYR-ASN-GLN-ARG-PHE-ASN-SER;

(vi) GLU-GLY-ILE-PHE-TYR-THR-MET-ASP-TYR.

The invention also relates to nucleic acid sequences coding for such polypeptides, to pharmaceutical compositions comprising such polypeptides and to immuneragents containing such polypeptides.

## Anti-hCG antibodies

The invention relates to polypeptides with immune reactivity in respect of hCG, hCG-binding fragments thereof, nucleic acids which are coding for this or contain a sequence coding for this, host cells which contain these nucleic acids and pharmaceutical products and immune reagents which contain the said polypeptides and/or fragments.

For birth control in humans use is frequently made of pharmaceuticals, which are usually administered to the female. The active substances in these products intervene in one or more of the biological processes which play a role in reproduction.

For many decades already it has been in the main steroids, which influence ovulation, which have been used for this purpose.

During the past decade attention has also been directed towards influencing other crucial phases of the reproduction process and specifically to the processes in which the hormone hCG (human chorion gonadotrophin) plays a role. hCG is a glycoprotein hormone which, inter alia, is secreted by the trophoblastic tissue of the placenta, and, after binding to specific membrane receptors, stimulates the production of steroid hormones. The steroid hormones produced (inter alia progesterone) are responsible for maintaining the pregnancy. Suppression of the action of the hCG can, thus, lead to abortion of the pregnancy.

It has been attempted to suppress the action of hCG by means of immunological methods. For this purpose, hCG was administered which was modified such that an immune reaction could be expected. This approach has not proved successful to date; in general, the antibody production was found to be too low or the antibodies were found to lack the desired specificity to capture the hCG sufficiently.

For this reason another approach has now been chosen with which the hCG concentration can be successfully lowered by administering polypeptides (such as antibodies) which react specifically with hCG.

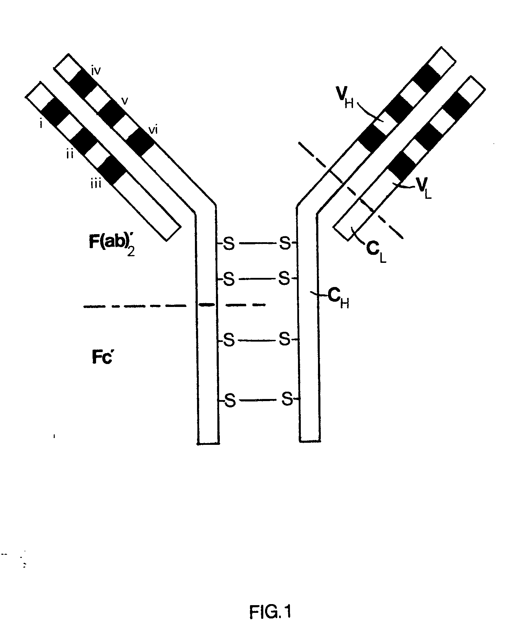

Naturally occurring antibodies (immunoglobulins; Ig) are differentiated into various types which, however, have in common that they all possess at least two antigen-binding (Fab) regions. The types are differentiated by differences in the remaining (Fc) regions of the protein. In the main the following types are to be differentiated: IgA, IgD, IgE, IgG and IgM; within these categories, sub-types are also frequently further distinguished. The structure of the IgG molecule, and the fine structure which will be discussed below, are shown schematically in Figure 1. The antibody molecules are made up of light and heavy polypeptide chains, designated L and H respectively. The regions of the respective polypeptide chains which have amino end groups are involved in the specific binding of antigens. These regions are variable to a significant degree and they are, in fact, unique for each antibody directed against a specific part (epitope) of an antigen. They are designated LFv and HFv respectively and together form the Fv region of an antibody molecule. The regions of the chains which have carboxyl end groups are less variable; i.e. they show only differences which are characteristic for the various types and sub-types, already mentioned above, which are distinguished in the antibodies. They are designated LFc and HFc respectively.

The Fc regions of corresponding antibody types of different animal species frequently show appreciable differences in amino acid composition and sequence.

Accordingly, antibodies from another animal species will be recognized as foreign and give rise to an immune rejection reaction.

Recent studies have shown that the Fv regions themselves consist of more and less variable regions. The highly variable (hypervariable) regions are found to be specifically responsible for the antigen binding and are designated CDR's (complementarity determining regions). On each of the chains there are three CDR's, embedded in the so-called FR's ("framework regions"). The function of the latter is probably to maintain the spatial structure for the correct positions of the CDR's. The six CDR's of the Fv region together form the total contact site of the antibody with the antigen against which it is directed.

In view of the detailed knowledge, summarized above, of the fine structure of immunoglobulin molecules and of the functions ascribed to the various sub-structures, it is in principle possible purposefully to make modifications to these molecules. Recombinant DNA techniques are, specifically, highly suitable for this purpose and a number of practical applications have been described for genetic manipulation techniques of this type.

European Patent Publication 0 088 994 of Schering Corp. describes expression vectors which contain DNA sequences which code for LFv or HFv fragments of a specific immunoglobulin, and expression of such immunoglobulin fragments in Escherichia coli (E. coli).

EP 0 120 694 of Celltech Ltd. relates, inter alia, to expression in one host cell of both light and heavy chains of an immunoglobulin or Fv fragments thereof. The immunoglobulins prepared in this way can, if desired, consist of a Fv region identical to that of a specific monoclonal antibody and a Fc fragment from another antibody (so-called chimeric immunoglobulin).

EP 0 125 023 of Genentech and City of Hope relates, like the two above patent publications, to the expression in a host cell of DNA which codes for an immunoglobulin fragment, or a chimeric immunoglobulin. The host cell here is a procaryotic or eucaryotic microorganism.

In EP 0171 496 of the Research Development Corporation of Japan the preparation of chimeric antibodies with the aid of recombinant DNA techniques is likewise described, in which, specifically, the Fc region corresponds to that of a non-human immunoglobulin.

WO 86/01533 of Celltech Ltd. is specifically restricted to the expression in immortalized mammal cells of chimeric proteins with an antigen-binding region and another protein region which, for example, can be a Fc region of another immunoglobulin but may also be (a part of) another protein (enzyme for example).

EP 0 029 400 of Winter describes the preparation of modified antibodies in which at least some of the CDR's of the Fv region have been replaced by corresponding CDR'(s) from another immunoglobulin. By means of this latter technique (designated "CDR grafting") it is possible to transfer CDR's from, for example, a mouse monoclonal immunoglobulin into a molecule which is otherwise characteristic of human immunoglobulin.

The aim of the present invention is to provide polypeptides (especially immunoglobulins or fragments thereof) which possess a particular specific affinity in respect of hCG.

The polypeptides according to the invention can, in particular, be employed for specific recognition of hCG in the cases in which the risk of cross reaction of the polypeptides with substances similar to hCG is high. This relates specifically to cross reaction with proteins such as human luteinising hormone (hLH) and human follicle-stimulating hormone (hFSH), which each, like hCG, consist of two polypeptide chains, one of which (the $\alpha$ chain) is identical to that of hCG and the other (the $\beta$ chain) displays a more or less strong homology with the $\beta$ chain of hCG.

An attempt has therefore been made to find antibodies which are tolerated by the human body and which have a very strong affinity for hCG but which bind the proteins homologous with hCG to a much lesser degree. In particular, suitable antibodies have been found to be polypeptides which possess one or more of the following CDR's:

(i) ARG-ALA-SER-GLU-SER-VAL-ASP-ASN-ASN-GLY-ILE-SER-PHE-MET-ASN;

(ii) ALA-ALA-SER-ILE-GLN-GLY-SER;

(iii) GLN-GLN-ILE-LYS-GLU-VAL-PRO-PRO-THR;

(iv) ASP-TYR-ASN-MET-HIS;

(v) TYR-ILE-TYR-PRO-TYR-SER-GLY-PRO-THR-GLY-TYR-ASN-GLN-ARG-PHE-ASN-SER;

(vi) GLU-GLY-ILE-PHE-TYR-THR-MET-ASP-TYR.

Of course, CDR's which functionally correspond to the abovementioned CDR's and which, for example, differ from the abovementioned CDR's in that one or more amino acids have been replaced or derivatized without this resulting in disturbance of the specific spatial structure of the CDR are likewise suitable for this application.

The polypeptides according to the invention can contain one or more of the above mentioned CDR's or functional equivalents thereof, preferably together with naturally occurring and/or synthetically produced FR's.

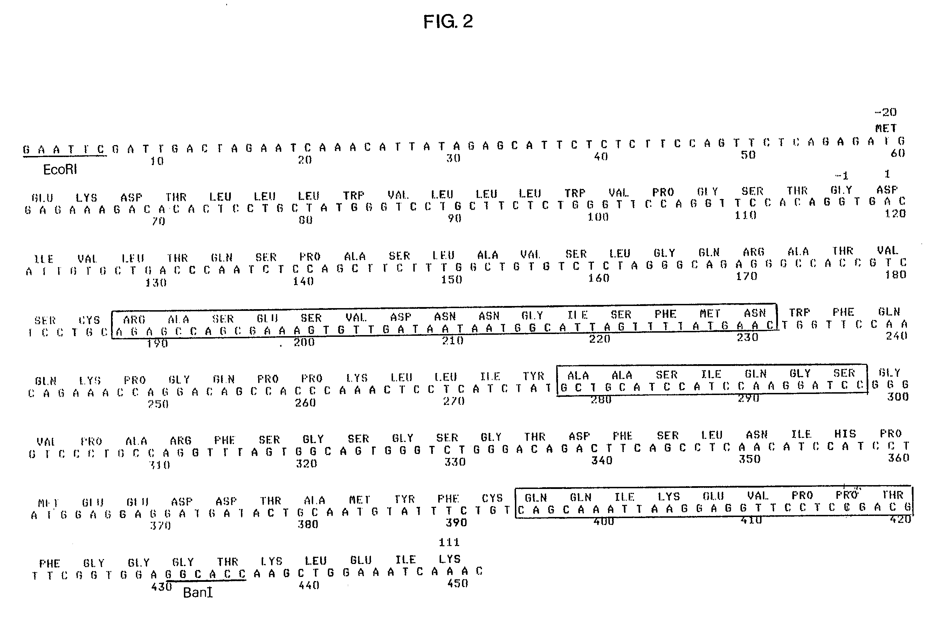

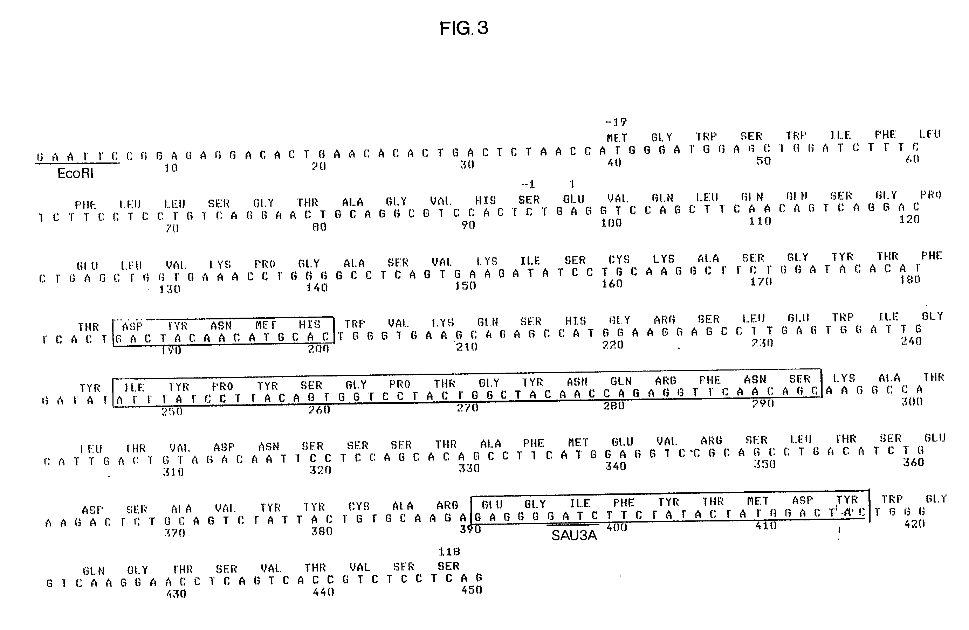

The sequences of the N-terminal regions of, respectively, the light and the heavy chain of a polypeptide according to the invention are shown in Figures 2 and 3, which N-terminal regions together form a hCG-binding antibody fragment (FhCG). The nucleotide sequence coding for these amino acid sequences are also shown in these figures. The CDR's are boxed both in the amino acid sequence and in the nucleotide sequence in Figures 2 and 3.

In place of the FR's shown in these Figures 2 and 3, other naturally occurring FR's or amino acid sequences with the same function produced from naturally occurring FR's can also be used. For example, these will be sequences which correspond to FR's of human antibodies.

The hCG-binding antibody fragments FhCG can be used as such or can be incorporated in a larger polypeptide.

For this purpose two or more of the said FhCG polypeptides can be coupled to one another - preferably by mutual covalent bonds or by means of a support. Another possibility is that at least part of a Fc fragment of an antibody chain is covalently coupled (possibly via a so-called linker) to the C-terminus of one or both Fv fragments.

It is also possible that one or both Fv fragments is coupled via the C-terminus to (parts of) other polypeptides, which preferably do not cause an immune reaction in the organism to which the polypeptide is administered.

According to the present invention the polypeptides are prepared, for example, with the aid of recombinant DNA methods. For this purpose a nucleotide sequence which codes for the intended polypeptide is inserted by known methods into a vector which is suitable for stable transformation of a chosen host cell, this host cell acquiring the ability to produce said polypeptide. In a vector of this type the intended nucleotide sequence is incorporated in such a way that control elements for expression thereof (transcription systems) are functionally connected thereto. At the same time said vectors can contain elements by means of which replication of the functionally relevant part thereof in the host cell is ensured. For this purpose the vector can contain its own replicon, such as the origin of replication in E. coli, or the SV40 origin of replication, which can be used in the case of eucaryotic cells. Eucaryotic cells can also be transformed with a vector which integrates in a stable manner in the chromosome of the host cell.

Host cells which can be used are procaryotic or eucaryotic cells and in particular mammal cells -preferably the latter in view of the fact that these may also be able to effect post-translational treatments, such as glycosylation and assemblage, on the polypeptide.

Host cells of this type transformed with a suitable vector which contains a nucleotide sequence coding for the polypeptide according to the invention can be cultured in vitro and under suitable conditions produce, and preferably secrete, the intended hybrid polypeptide. The culture systems and culture conditions to be used for this purpose are dependent on the host cell chosen. Preferably, the conditions will be chosen such that the desired polypeptide is secreted into the culture medium, from which it can be further isolated if appropriate.

The polypeptides ·according to the invention can be used to prevent pregnancies, but also for other therapeutic purposes, such as for combatting choriocarcinomas or other hCG-producing tumours. The hCG-binding polypeptides can also be used for diagnostic applications, such as for in vivo diagnostics (for example for localization of tumours) and for in vitro diagnostics, for the detection of hCG in body fluids (blood, serum, urine and the like).

For therapeutic purposes the polypeptides according to the invention are administered incorporated into a therapeutically acceptable carrier. Such a carrier contains, specifically, water and also possibly a pH-buffering compound, stabilizers, antibiotics, carbohydrates, glycine, benzyl alcohol and the like.

For combatting choriocarcinomas, the polypeptides according to the invention can be administered together with, and possibly coupled to, substances which can inhibit or stop the tumour development and/or can kill or help to kill the cells of the tumour. For this purpose the polypeptides can, for example, be coupled to cytostatic or cytotoxic substances.

In the case of the in vitro diagnosis of hCG, the hCG-binding polypeptide can, for example, be bound to a particle, to a solid support or to a marker.

Example 1

This example describes the production of a hybrid antibody of which the variable parts (V) originate from mice and the constant parts (C) from humans. Genes were constructed, coding for both the light and the heavy chain, based on hereditary material from both species.

The genes coding for the variable fragments were isolated from an anti-hCG-producing myeloma cell line. RNA was isolated from this and cDNA prepared (Gubler, U., and Hoffmann, B.J., Gene 25, 263-269, 1983). Using known molecular-biological methods (Maniatis, T., Fritsch, E.T. and Sambrook, J., Molecular Cloning: a laboratory manual. Cold Spring Harbor Laboratory, Cold Spring Harbor, New York, 1982), a cDNA library was constructed in bacteriophage $x$gt10. Clones were selected by hybridization with probes which are derived from the already known nucleotide sequence of the two constant regions ($\gamma_1$ and $x$ ) (Honjo, T., Obata. M., Yamawaki-Kataoka, Y., Katoaka, T., Kawakami, T., Takahashi, N. and Mano, Y., Cell 18, 559-568, 1979; Max. E.E., Maizel, J.V. and Leder, P., J. Biol. Chem. 256, 5116-5120, 1981).

The inserts of the clones obtained in this way were then transferred to bacteriophage M13 and the nucleotide sequence of the variable region (L and H chain) determined using the dideoxy method (Sanger, F., Nicklen, S. and Coulson, A.R., Proc.Natl.Acad.Sci. USA 74, 5463-5467, 1977) (Fig. 2 and 3 respectively). From this sequence it was furthermore possible to derive the amino acid sequence and the position of the CDR's.

By using the nucleotide sequences of these V regions and those of human C regions already published earlier, hybrid H and L genes were constructed.

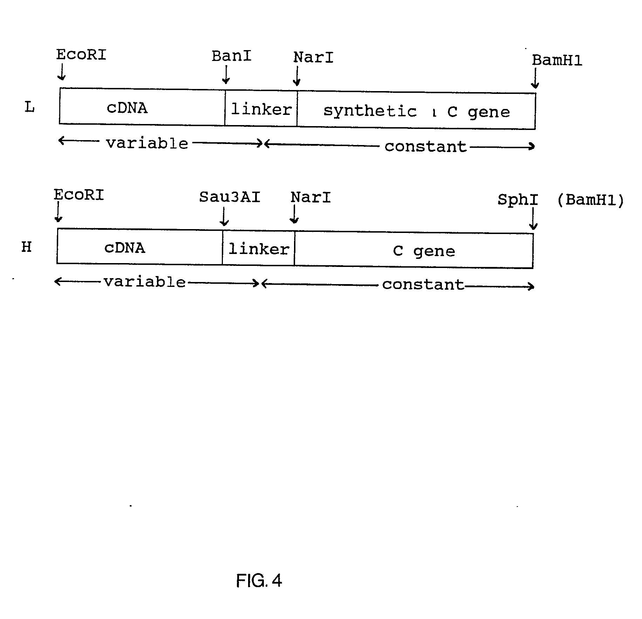

Figure 4 shows a schematic representation of these genes.

The variable fragments were restricted both on the 5'- and on the 3'-side with the aid of restriction enzyme recognition sites. For this purpose, a recognition site for the enzyme EcoRI was created on the 5'-side, shortly before the position of the AUG-initiation codon, by addition of EcoRI linkers (nucleotide positions 1-6 in Figures 2 and 3). On the 3'-side use was made of the recognition sites already present for the enzymes Sau3AI (56 nucleotides before the end of the variable H region) and BanI (22 nucleotides before the end of the variable L region). The EcoRI-Sau3AI and EcoRI-BanI fragments obtained in this way code for the virtually complete variable region of the H and L gene respectively.

The gene coding for the constant H region is derived from a library of human foetal liver DNA (Takahashi, N., Neda, S., Obata, M., Nikaido, T., Nakai, S. and Honjo, T., Cell 29, 671-679, 1982). A$\gamma$2 gene was chosen. The gene was isolated as a NarI-SphI DNA fragment of approximately 2200 base pairs. The NarI recognition site is located at position 20 of the first exon (CH1); the SphI recognition site lies approximately 650 base pairs away from the stop codon, so that with the exception of the first 20 bases the complete $C_H$ sequence is located on this DNA fragment. Moreover, 3 introns and the natural polyadenylation signal (Ellison, J. and Hood, L., Proc.Natl.Acad.Sci. USA, 79, 1984-1988, 1982) are located on this fragment.

The gene coding for the human $x$ constant part is prepared via oligonucleotide synthesis as a NarI-BamHI fragment of 315 base pairs. The NarI recognition site is created at position 11 of the coding $C_x$ part. In addition, a Pst I recognition site was created halfway along the gene, as a result of which the synthesis was able to take place in two halves. These two recognition sites were chosen such that the amino acid sequence for which the nucleotide sequence codes (Hieter, P.A., Max, E.E., Seidman, J.G., Maizel, J.V. and Leder, P., Cell 22, 197-207, 1980) did not undergo any modifications. In comparison with the sequence of Hieter et al, other bases were introduced in 4 positions, i.e. position 344, T → G; 347, A → G; 473, c → G and 476, A → G (numbering from Hieter et al., above).

The BamHI recognition site at the end of the fragment partially overlaps with the stop codon (5′-TAGG TATCC-3′). The synthesis is carried out in two blocks of 5 and 6 oligonucleotides. Some of the oligonucleotides from one block are prepared by complementary synthesis and are linked to one another with the aid of the enzyme $T_4$-DNA-ligase and inserted in the bacteriophage M13. After checking the nucleotide sequence, both blocks are connected together to form the NarI-BamHI $C_x$ fragment. The synthesis of the oligonucleotides was carried out on an Applied Biosystem 318A DNA synthesizer.

In order to link the two V and C fragments of both the H and L gene to one another, DNA fragments (termed linkers in Figure 4) were designed (Sau3AI-NarI and BanI-NarI respectively) which code for the final amino acids of V and (consecutively) for the first amino acids of C. These DNA fragments were again chemically synthesized. The chimeric gene was obtained by coupling the V and C fragments via the linking fragment, which for each of the two chains coded for the original amino acid sequence of the relevant respective chain fragments, and in addition as far as possible contained the original base sequence ("linker" in Figure 4). The DNA fragments thus obtained code for an immunoglobulin chain in which the amino acid sequence of the V region is wholly derived from mice and the C region from humans. The nucleotide sequences are, as described above, for the mouse V and human C region.

In order to facilitate insertion in an expression vector, the SphI recognition site of the H gene was modified to a BamHI recognition site by linker addition, so that both genes are now located on a EcoRI-BamHI fragment.

The vector which is used for expression in eucaryotic cells is largely derived from pKCR (O'Hare, K., Benoist, C and Breathnach, R., Proc.Natl.Acad.Sci. USA 78, 1527-1531, 1981).

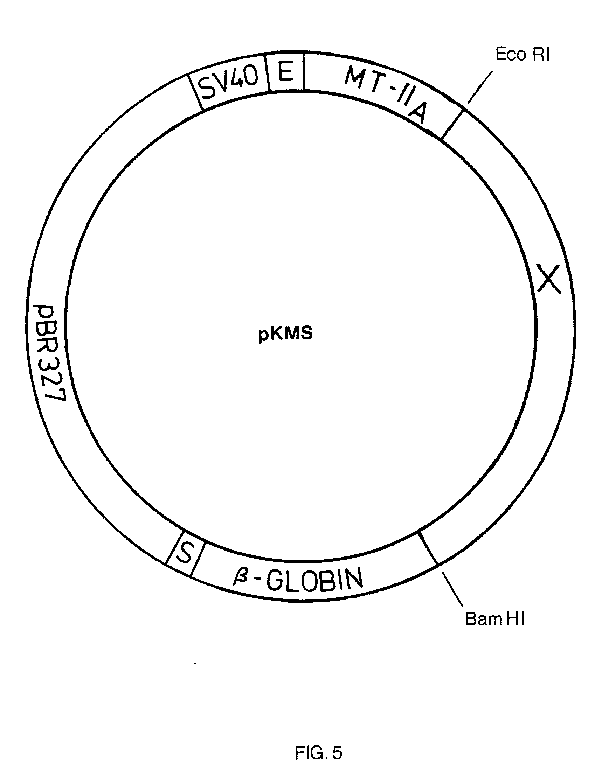

The vector, termed pKMS, is shown in Figure 5 and consists of the following elements:

a. human metallothionine-II$_A$ promoter (MT-II$_A$) from which transcription of the inserted gene (X, is H or L gene) takes place. The promoter is located on an 837 base pair Hind-III-NcoI fragment (-764 and +73 with respect to the transcription start; Karen, M. and Richards, R.I., Nature 299, 797-802, 1982), the NcoI recognition site of which has been modified to a EcoRI site. For this purpose the NcoI recognition site was first removed with the aid of the enzyme Mung Bean nuclease. The MT-II$_A$ fragment was then inserted in MI3mp11 (HindIII x SstI, the SstI side having been rendered "blunt" by digestion with Mung Bean nuclease). The MT-II$_A$ fragment was then isolated as a EcoRI-HindIII-DNA fragment from M13mp11-ds-DNA:

b. MuLV(E)- and SV40-enhancer sequences which serve to enable the transcription to be as high as possible. The retroviral enhancer is derived from the LTR of MuLV and consists of a Sau3AI-XbaI DNA fragment 203 base pairs in size (Van Beveren, C., Rands, E., Chattopadhyay, S.K., Lowy, D.R. and Verma, I.M., J.Virol. 41, 542-556, 1982). The SV40-DNA fragment is 326 base pairs long and contains the early promoter (HindIII-PvuII fragment derived from the plasmid pSV2CAT, Gorman, C.M., Moffat, L.F and Howard, B.H., Molec.Cell.Biol. 2, 1044-1051, 1982). The XbaI recognition site of the MuLV-enhancer was originally linked to the EcoRI recognition site of pBR322 (EcoRI recognition site rendered "blunt" by digestion with Mung Bean nuclease). The HindIII recognition site of pBR322 was then used for attachment of the MT-II$_A$ promoter, so that a 29 base pair "EcoRI"-HindIII fragment derived from pBR332 is located between the MuLV enhancer and the MT-II$_A$ promoter. A unique C1aI recognition site is located on this fragment.

The SauIIIA recognition site of the MuLV enhancer was rendered blunt by filling with the aid of DNA polymerase I and attached to the PvuII side of the SV40 promoter;

c. the final (coding) part of the rabbit $\beta$-globin gene and the SV40 transcription terminator by means of which intronless genes (such as the

chimeric x-gene) are provided with an intron on the primary messenger RNA and with a terminator;

d. pBR327 sequence with the E.coli replication starting point and ampicillin-resistance gene for replication and selection in E.coli. The last-mentioned constituents and also the region mentioned under c are derived from the largest BamHI-EcoRI fragment of plasmid pKCR (O'Hare, K., Benoist, C. and Breathnach, R., Proc.Natl.Acad.Sci. USA 78, 1527-1531, 1981) with the proviso that the so-called "poisonous sequence" of pBR322 has been removed by exchange with a homologous region of pBR327 and that the final coding $\beta$-globin-exon has been largely removed (base pairs 1122-1196 out: van Ooyen, A., van den Berg, J., Mantel, N. and Weissmann, C., Science 206, 337-344, 1979). This latter was effected by partial digestion with the enzymes EcoRI and BgIII for which recognition sites are present in the $\beta$-globin gene and filling the "sticky ends" with DNA-polymerase I. The two ends were then linked to one another.

A second EcoRI recognition site present in pKCR was removed by filling with DNA polymerase. During the construction of pKMS the following elements were positioned (in the correct sequence) between this filled EcoRI recognition site and the HindIII recognition site of the SV40 promoter:
- EcoRI (filled) -ClaI-DNA fragment (24 base pairs) derived from pBR322;
- a region of the polylinker: HindII - HindIII-DNA fragment (17 base pairs) derived from M13mp10.

The chimeric H and L genes were each separately inserted between the EcoRI and BamHI splitting sites of pKMS. The plasmids thus formed, termed pKMS.H and pKMS.L, were used to transfect Chinese Hamster Ovary cells (CHO.K1; ATCC CCL-61) with the aid of the Ca phosphate precipitation method (Wigler, M., Pellicer, A., Silverstein, S., Axel, R., Urlaub, G. and Chasin, L., Proc.Natl.Acad.Sci. USA 76, 1373-1376, 1979). For this purpose a DNA mixture was used consisting of 10.6 $\mu$g pKMS.H, 8.2 $\mu$g pKMS.L and 1.2 $\mu$g of a plasmid containing the neomycin-resistance gene and the MT-II$_A$ gene providing resistance against heavy metals (pAG60, ColbèreGarapin, F., Horodinceau, F., Kourilsky, P. and Garpin, A.C., J.Mol.Biol. 150, 1-14, 1981) with a 3 kbp HindIII-DNA fragment containing the human MT-II$_A$ gene (Karin, M. and Richards, R.I., Nature 299, 797-802, 1982) integrated in the HindIII recognition site. 48 hours after transfection the selection was started by refreshing the growth medium with + G418 medium in a concentration of 0.8 mg/ml. After approximately 14 days a second selection procedure was started. For this purpose the cell population selected for the presence of the neomycin gene was split into five parts, to which growth medium in the presence of

50 mM $ZnCl_2$ and an increase in concentration of $CdCl_2$ (1-5 $\mu$m) was added. A selection of cells which can bring the MT-II$_A$ gene to expression well takes place by this means. Because the immunoglobulin plasmids are transfected at the same time as the selection plasmid and probably are integrated at the same site in the chromosomal DNA it is likely that in cells where a high expression of MT-II$_A$ gene takes place the immunoglobin genes will also be brought to expression well. Supernatant of a specific cell population selected with $Cd^{2+}$ was then tested for the presence of chimeric antibodies. Cells from a $Cd^{2+}$- resistant cell population with the highest antibody production were cloned. The test to detect antibodies is based on a "sandwich" EIA, the sample being introduced into a well of a microtitre plate to which goat anti-human IgG is attached. After washing out, the plate with the sample was incubated with a conjugate of goat serum against human j and horse radish peroxidase. Coupling of enzyme-bound antibody to the well was detected after additions of the substrate TMB by a colour reaction. A similar test was carried out with hCG bound to the plate and with goat serum directed against human Ig coupled to HRP as conjugate. G418-resistant cell populations were reproducibly positive in both tests. This means that the CHO.K1 cells transfected with the abovementioned plasmids are capable of secreting a protein which consists of both a light and a heavy human immunoglobulin chain. In addition, the protein can bind to hCG.

## Example 2

The second example describes the contruction of genes encoding a hybrid antibody in which only part of the variable regions i.e. the CDR's are derived from the mouse whereas the FR's and the constant parts are of human origin. The variable heavy and light chain gene sequences have been synthesized chemically. The genes were designed in such a way that the CDR regions are identical to the boxed sequences in fig. 2 and 3 whereas the FR regions are derived from a human antibody called WEA (Goñi, F., and Frangione, B., Proc. Natl. Acad. Sci. USA 80, 4837-4841, 1983). The signal sequences are derived from consensus sequences for the $V_{xI}$ and $V_{HIII}$ gene families. The WEA antibody chains are representatives of these families. Fifty eight oligodeoxyribonucleotides ranging in size from 25-48 nucleotides were synthesized and assembled in 9 sections. The 5'-termini of the oligonucleotides in one section were 5'-phosphorylated (with exception of the two outmost oligonucleotides), annealed and ligated into M13mp11 RF DNA clones from all sections were selected by sequence analysis. DNA fragments were purified by polyacrylamide gel electrophoresis and they were ligated to form the intact variable region sequences. Construction of the complete immunoglobulin genes, insertion into the vector and transfection of Chinese Hamster Ovary cells were as described in previous the example.

## Claims

1. Polypeptide with binding-activity for hCG, characterized in that it contains at least one of the polypeptide fragments:

(i) ARG-ALA-SER-GLU-SER-VAL-ASP-ASN-ASN-GLY-ILE-SER-PHE-MET-ASN;

(ii) ALA-ALA-SER-ILE-GLN-GLY-SER;

(iii) GLN-GLN-ILE-LYS-GLU-VAL-PRO-PRO-THR;

(iv) ASP-TYR-ASN-MET-HIS;

(v) TYR-ILE-TYR-PRO-TYR-SER-GLY-PRO-THR-GLY-TYR-ASN-GLN-ARG-PHE-ASN-SER;

(vi) GLU-GLY-ILE-PHE-TYR-THR-MET-ASP-TYR.

2. Polypeptide which contains at least one region of an immunoglobulin chain, with antigen- binding domains which are mutually linked by so- called framework regions, characterized in that it contains at least one antigen-binding domain comprising one of the polypeptide fragments:

(i) ARG-ALA-SER-GLU-SER-VAL-ASP-ASN-ASN-GLY-ILE-SER-PHE-MET-ASN;

(ii) ALA-ALA-SER-ILE-GLN-GLY-SER;

(iii) GLN-GLN-ILE-LYS-GLU-VAL-PRO-PRO-THR;

(iv) ASP-TYR-ASN-MET-HIS;

(v) TYR-ILE-TYR-PRO-TYR-SER-GLY-PRO-THR-GLY-TYR-ASN-GLN-ARG-PHE-ASN-SER;

(vi) GLU-GLY-ILE-PHE-TYR-THR-MET-ASP-TYR.

3. DNA sequence, characterized in that it codes for a polypeptide according to claim 1-2.

4. DNA sequence, characterized in that a region thereof codes for a polypeptide according to claim 1-2.

5. Recombinant DNA, characterized in that it contains a nucleotide sequence according to claim 4.

6. Expression vector which contains a DNA sequence according to claim 4, under the direction of expression control elements.

7. Host cell which has been transformed with an expression vector according to claim 4 and which is capable of producing a polypeptide according to claim 1.

8. Pharmaceutical preparation containing a polypeptide according to claim 1-2.

9. Immune reagent for diagnostic or therapeutic application containing a polypeptide according to claim 1-2.

10. Immune reagent according to claim 9, characterized in that it is bonded to an insoluble support, a particle, a marker or a cytotoxic substance.

FIG.1

FIG. 2

EP 0 370 581 A1

```
                                                                                    -20
                                                                                    MET
G A A T T C G A T T G A C T A G A A T C A A A C A T T A T A G A G C A T T C T C T C T T C C A G T T C T C A G A G A T G
         10                  20                  30                  40                  50                  60
    EcoRI
                                                                                               -1       1
GLU   LYS   ASP   THR   LEU   LEU   LEU   TRP   VAL   LEU   LEU   LEU   TRP   VAL   PRO   GLY   SER   THR   GLY   ASP
G A G A A A G A C A C A C T C C T G C T A T G G G T C C T G C T T C T C T G G G T T C C A G G T T C C A C A G G T G A C
               70                  80                  90                 100                 110                 120

ILE   VAL   LEU   THR   GLN   SER   PRO   ALA   SER   LEU   ALA   VAL   SER   LEU   GLY   GLN   ARG   ALA   THR   VAL
A T T G T G C T G A C C C A A T C T C C A G C T T C T T T G G C T G T G T C T C T A G G G C A G A G G G C C A C C G T C
               130                 140                 150                 160                 170                 180

SER   CYS   ARG   ALA   SER   GLU   SER   VAL   ASP   ASN   ASN   GLY   ILE   SER   PHE   MET   ASN   TRP   PHE   GLN
T C C T G C A G A G C C A G C G A A A G T G T T G A T A A T A A T G G C A T T A G T T T T A T G A A C T G G T T C C A A
               190               .   200                 210                 220                 230                 240

GLN   LYS   PRO   GLY   GLN   PRO   PRO   LYS   LEU   LEU   ILE   TYR   ALA   ALA   SER   ILE   GLN   GLY   SER   GLY
C A G A A A C C A G G A C A G C C A C C C A A A C T C C T C A T C T A T G C T G C A T C C A T C C A A G G A T C C G G G
               250                 260                 270                 280                 290                 300

VAL   PRO   ALA   ARG   PHE   SER   GLY   SER   GLY   SER   GLY   THR   ASP   PHE   SER   LEU   ASN   ILE   HIS   PRO
G T C C C T G C C A G G T T T A G T G G C A G T G G G T C T G G G A C A G A C T T C A G C C T C A A C A T C C A T C C T
               310                 320                 330                 340                 350                 360

MET   GLU   GLU   ASP   ASP   THR   ALA   MET   TYR   PHE   CYS   GLN   GLN   ILE   LYS   GLU   VAL   PRO   PRO   THR
A T G G A G G A G G A T G A T A C T G C A A T G T A T T T C T G T C A G C A A A T T A A G G A G G T T C C T C C G A C G
               370                 380                 390                 400                 410                 420

                                                      111
PHE   GLY   GLY   GLY   THR   LYS   LEU   GLU   ILE   LYS
T T C G G T G G A G G C A C C A A G C T G G A A A T C A A A C
               430   BanI           440                 450
```

FIG. 3

```
                                                                    -19
                                                           MET  GLY  TRP  SER  TRP  ILE  PHE  LEU
GAATTCCGGAGAGGACACTGAACACACTGACTCTAACCATGGGATGGAGCTGGATCTTTC
         10        20         30         40        50          60
EcoRI

                                                         -1    1
   PHE  LEU  LEU  SER  GLY  THR  ALA  GLY  VAL  HIS  SER  GLU  VAL  GLN  LEU  GLN  GLN  SER  GLY  PRO
TCTTCCTCCTGTCAGGAACTGCAGGCGTCCACTCTGAGGTCCAGCTTCAACAGTCAGGAC
       70          80          90        100        110         120

   GLU  LEU  VAL  LYS  PRO  GLY  ALA  SER  VAL  LYS  ILE  SER  CYS  LYS  ALA  SER  GLY  TYR  THR  PHE
CTGAGCTGGTGAAACCTGGGGCCTCAGTGAAGATATCCTGCAAGGCTTCTGGATACACAT
         130        140         150        160        170         180

   THR  ASP  TYR  ASN  MET  HIS  TRP  VAL  LYS  GLN  SER  HIS  GLY  ARG  SER  LEU  GLU  TRP  ILE  GLY
TCACTGACTACAACATGCACTGGGTGAAGCAGAGCCATGGAAGGAGCCTTGAGTGGATTG
         190        200         210        220         230         240

   TYR  ILE  TYR  PRO  TYR  SER  GLY  PRO  THR  GLY  TYR  ASN  GLN  ARG  PHE  ASN  SER  LYS  ALA  THR
GATATATTTATCCTTACAGTGGTCCTACTGGCTACAACCAGAGGTTCAACAGCAAGGCCA
         250        260        270         280         290         300

   LEU  THR  VAL  ASP  ASN  SER  SER  SER  THR  ALA  PHE  MET  GLU  VAL  ARG  SER  LEU  THR  SER  GLU
CATTGACTGTAGACAATTCCTCCAGCACAGCCTTCATGGAGGTCCGCAGCCTGACATCTG
         310        320         330        340         350         360

   ASP  SER  ALA  VAL  TYR  TYR  CYS  ALA  ARG  GLU  GLY  ILE  PHE  TYR  THR  MET  ASP  TYR  TRP  GLY
AAGACTCTGCAGTCTATTACTGTGCAAGAGAGGGGATCTTCTATACTATGGACTACTGGG
         370        380         390         400        410         420
                                    SAU3A

                                             118
   GLN  GLY  THR  SER  VAL  THR  VAL  SER  SER
GTCAAGGAACCTCAGTCACCGTCTCCTCAG
         430        440        450
```

FIG. 4

FIG. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 800 974  (WHITEHEAD INSTITUTE FOR BIOMEDICAL RESEARCH) <br> * Pages 0,1,30-31; figure 1 * & <br> EP-A-316 354 <br> --- | 1-3 | C 12 N 15/00 <br> A 61 K 37/00 <br> A 61 K 39/00 |
| A | WO-A-8 806 591  (SCRIPPS CLINIC AND RESEARCH FOUNDATION) <br> * pages 0-8,94-106; figures 2A-D * & <br> EP-A-305 488 <br> ----- | 1-3 | |

**TECHNICAL FIELDS SEARCHED (Int. Cl.5)**

C 07 K
A 61 K

The present search report has been drawn up for all claims

| Place of search | Date of completion of the search | Examiner |
|---|---|---|
| THE HAGUE | 05-02-1990 | RAJIC M. |